# EUROPÄISCHE PATENTSCHRIFT

(11) **EP 1 053 216 B1**
(45) Veröffentlichungstag und Bekanntmachung des Hinweises auf die Patenterteilung: **10.09.2003**
(21) Anmeldenummer: 99904831.7
(22) Anmeldetag: 30.01.1999
(51) Int. Cl.: C07C 13/72

(54) **SPIROVERBINDUNGEN UND DEREN VERWENDUNG**
SPIRANS AND THE USE THEREOF
SPIRANNES ET LEUR UTILISATION

(30) Priorität: 04.02.1998 DE 19804310
(43) Veröffentlichungstag der Anmeldung: 22.11.2000
(73) Patentinhaber: Covion Organic Semiconductors GmbH, 65926 Frankfurt (DE)
(72) Erfinder: WEISSÖRTEL, Frank, D-65929 Frankfurt am Main (DE); SALBECK, Josef, D-65779 Kelkheim (DE); SPREITZER, Hubert, D-65929 Frankfurt am Main (DE)
(74) Vertreter: Luderschmidt, Schüler & Partner GbR
(86) Internationale Anmeldenummer: EP9900615
(87) Internationale Veröffentlichungsnummer: WO99040051

(56) Entgegenhaltungen:
- EP-A- 0 676 461
- WO-A-97/10617

## Beschreibung

Spiroverbindungen der allgemeinen Formel 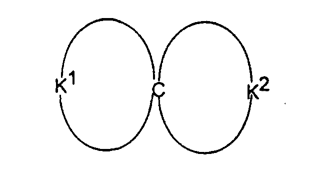 in der K¹ und K² unabhängig voneinander konjugierte Systeme bedeuten, haben beispielsweise als Elektrolumineszenzmaterialien (EP-A 0 676 461), als Ladungstransportschicht in photovoltaischen Zellen (WO-A 97/106 17), als Werkstoffe in der nichtlinearen Optik (EP-A 0 768 563) und als optische Aufheller (DE-A 196 45 063) Anwendung gefunden.

Es wurde nun überraschend gefunden, daß sich bestimmte Spiroverbindungen, die mehr als ein Spirozentrum enthalten, in besonderer Weise zur Verwendung als Laserfarbstoffe für organische Feststofflaser eignen.

Gegenstand der Erfindung sind daher Spiroverbindungen der Formel (I), 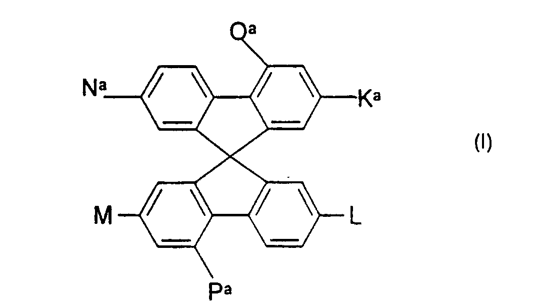 wobei K^{a}, L, M, N^{a}, O^{a}, P^{a} unabhängig voneinander gleich oder verschieden, 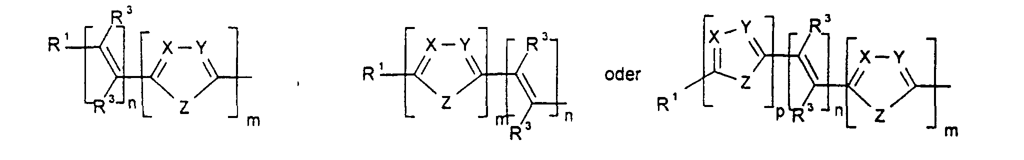 sind;
wobei die Symbole und Indizes die folgenden Bedeutungen haben:
- R³: H, C₁-C₂₂-Alkyl, CN, C₆-C₁₄-Aryl, C₄-C₁₅-Heteroaryl, C₅-C₂₀-Alkylaryl und C₅-C₂₀-Arylalkyl
- m, n, p:: unabhängig voneinander gleich oder verschieden 0, 1, 2, 3, 4, 5, oder 6;
- X, Y:: CR, N
- Z:: O, S, NR, CR₂, -CH=CH-, -CH=N-, -CR²=CR²- oder -CR²=N-;
- R :: H, C₁-C₂₂-Alkyl (linear, verzweigt oder cyclisch), C₁-C₂₂-Alkoxy, CN, NR²₂, COOR², CHO, SR², NO₂, OH, C₆-C₁₄-Aryl, wie Phenyl, Biphenyl, 1-Naphthyl, 2-Naphthyl, 9-Anthryl, C₄-C₁₅-Heteroaryl, vorzugsweise enthaltend ein, zwei oder drei N, O und/oder S Atome, besonders bevorzugt 2-Thienyl, 2-Furanyl, oder Aryloxy,
- R¹:: unabhängig voneinander, gleich oder verschieden R, A, B oder C ,
wobei 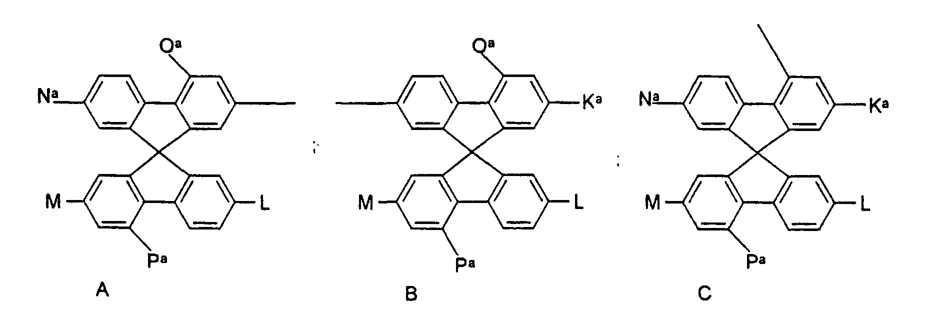 wobei mindestens ein, vorzugsweise 1 bis 10, Reste R¹ aus den Resten A, B oder C ausgewählt sind;
- R²: ist gleich oder verschieden H oder ein Kohlenwasserstoffrest mit 1 bis 30 C- Atomen, der auch ein oder mehrere, vorzugsweise ein, zwei oder drei, Heteroatome, vorzugsweise N, O und/oder S, enthalten kann,
ausgenommen Verbindungen der Formel (I) bei denen

| | |
|---|---|
| bei O^{a}, P^{a}, M und L gleichzeitig | R¹ ungleich A, B oder C ist |
| und zugleich bei K^{a} und N^{a} | R¹ gleich A oder B bedeutet. |

sowie Verbindungen der Formel (I) bei denen

| | |
|---|---|
| bei O^{a}, K^{a}, M und P^{a} gleichzeitig | R¹ ungleich A, B oder C ist |
| und zugleich bei N^{a} und L | R¹ gleich A oder B bedeutet. |

Bevorzugt sind Verbindungen der Formel (I), die dadurch gekennzeichnet sind, daß die Symbole und Indizes die folgenden Bedeutungen haben:
- n, p :: 0;
- m :: 0, 1, 2, 3, 4, 5 oder 6;
- X,Y :: CR;
- Z :: -CR=CR-; und
- R¹ :: unabhängig voneinander gleich oder verschieden R, A, B oder C, wobei mindestens ein Rest R¹ A, B oder C ist.
- R³ :: H, C₂-C₁₀-Alkyl, C₆-C₁₀-Aryl

Besonders bevorzugte Verbindungen der Formel (I) sind dadurch gekennzeichnet, daß bei K^{a}, L, M, N^{a} die Reste R¹ unabhängig voneinander gleich oder verschieden A, B oder C sind.

Ganz besonders bevorzugte Verbindungen der Formel (I) sind dadurch gekennzeichnet, daß O^{a} und P^{a} gleich H sind.

Ebenso ganz besonders bevorzugte Verbindungen der Formel (I) sind dadurch gekennzeichnet, daß R¹ gleich oder verschieden A, B oder C ist.

Ebenso besonders bevorzugte Verbindungen der Formel (I) sind dadurch gekennzeichnet, daß R¹ : R oder A ist, wobei mindestens ein R¹ gleich A ist.

Weiterhin ganz besonders bevorzugte Verbindungen der Formel (I) sind dadurch gekennzeichnet, daß R¹ gleich A ist.

Bevorzugt beträgt die Anzahl der Gruppen A, B und/oder C 1 bis 50, vorzugsweise bis 20, besonders bevorzugt 1 bis 10.

Verbindungen gemäß mindestens einem der vorhergehenden Ansprüche, dadurch gekennzeichnet, daß in Formel (I) O^{a} und P^{a} gleich H ist.

Insbesondere bevorzugte Verbindungen der Formel (I) sind solche der Formel (II), 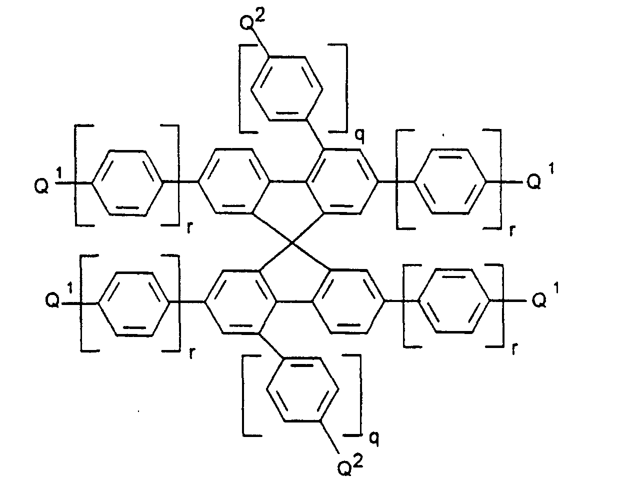 wobei die Symbole und Indizes die folgenden Bedeutungen haben:
- q,r:: unabhängig voneinander gleich oder verschieden 0, 1, 2, 3, 4, 5 oder 6;
- Q¹, Q²:: unabhängig voneinander gleich oder verschieden H oder 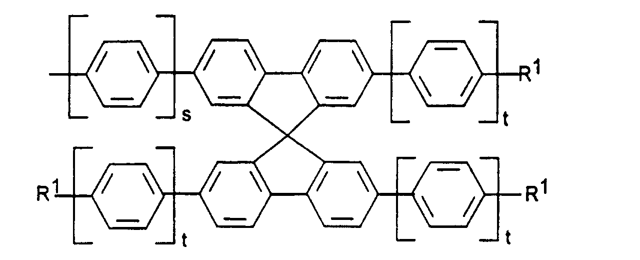
wobei
- s,t:: unabhängig voneinander gleich oder verschieden 0, 1, 2, 3 oder 4 ist; und
- R¹:: unabhängig voneinander gleich oder verschieden R bzw. A, B oder C ist und mindestens ein Rest Q¹ oder Q² ungleich Wasserstoff ist.

Bevorzugte Verbindungen gemäß Formel (II) sind dadurch gekennzeichnet, daß die Symbole und Indizes die folgenden Bedeutungen haben:
- Q¹, Q²:: unabhängig voneinander gleich oder verschieden H oder 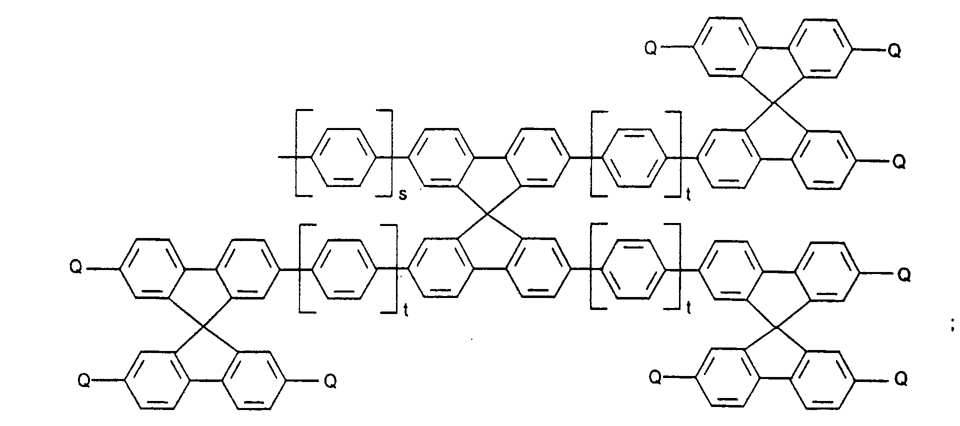
wobei
- s,t:: unabhängig voneinander gleich oder verschieden 0, 1, 2, 3 oder 4 ist; und
- Q:: unabhängig voneinander gleich oder verschieden R oder Q¹ ist.

Die Herstellung der erfindungsgemäßen Spiroverbindungen erfolgt nach an sich literaturbekannten Methoden, wie sie in Standardwerken zur Organischen Synthese, z.B. Houben-Weyl, Methoden der Organischen Chemie, Georg-Thieme-Verlag, Stuttgart und in den entsprechenden Bänden der Serie "The Chemistry of Heterocyclic Compounds" von A. Weissberger und E. C. Taylor (Herausgeber) beschrieben werden.

Die Herstellung erfolgt dabei unter Reaktionsbedingungen, die für die genannten Umsetzungen bekannt und geeignet sind. Dabei kann auch von an sich bekannten, hier nicht näher erwähnten Varianten Gebrauch gemacht werden.

Verbindungen der Formel (I) mit O^{a} = P^{a} = H werden beispielsweise ausgehend vom 9,9'-Spirobifluoren erhalten, dessen Synthese z.B. von R. G. Clarkson, M. Gomberg, J.Am.Chem.Soc. 52 (1930) 2881, beschrieben ist.

Die Herstellung von Verbindungen der Formel (I) mit K^{a} = L = M = N^{a}, O^{a} = P^{a} = H kann beispielsweise ausgehend von einer Tetrahalogenierung in den Positionen 2,2',7,7' des 9,9'-Spirobifluorens und anschließender Substitutionsreaktion erfolgen (siehe z.B. US 5,026,894) oder über eine Tetraacetylierung der Positionen 2,2',7,7' des 9,9'-Spirobifluorens mit anschließender C-C-Verknüpfung nach Umwandlung der Acetylgruppen in Aldehydgruppen oder Heterocyclenaufbau nach Umwandlung der Acetylgruppen in Carbonsäuregruppen erfolgen.

Die Herstellung von Verbindungen der Formel (I) mit K^{a} = M = P^{a} = O^{a} = H und N^{a} = L kann beispielsweise dadurch erfolgen, daß die stöchiometrischen Verhältnisse bei der Umsetzung so gewählt werden, daß die Positionen 2,2' bzw. 7,7' funktionalisiert werden (siehe z.B. J. H. Weisburger, E. K. Weisburger, F. E. Ray, J. Am. Chem. Soc. 72 (1959) 4253; F. K. Sutcliffe, H. M. Shahidi, D. Paterson, J. Soc. Dyers Colour 94 (1978) 306 und G. Haas, V. Prelog, Helv. Chim. Acta 52 (1969) 1202).

Für die Synthese der Gruppen K^{a}, L, M, N^{a}, sei beispielsweise verwiesen auf DE-A 23 44 732, 24 50 088, 24 29 093, 25 02 904, 26 36 684, 27 01 591 und 27 52 975 für Verbindungen mit 1,4-Phenylen-Gruppen;
DE-A 26 41 724 für Verbindungen mit Pyrimidin-2,5-diyl-Gruppen;
DE-A 40 26 223 und EP-A 03 91 203 für Verbindungen mit Pyridin-2,5-diyl-Gruppen;
DE-A 32 31 462 für Verbindungen mit Pyridazin-3,6-diyl-Gruppen; N. Miyaura, T. Yanagi und A. Suzuki in Synthetic Communications 11 (1981) 513 bis 519, DE-A-3 930 663, M. J. Sharp, W. Cheng, V. Snieckus in Tetrahedron Letters 28 (1987), 5093; G. W. Gray in J. Chem. Soc. Perkin Trans II (1989) 2041 und Mol. Cryst. Liq. Cryst. 172 (1989) 165, Mol. Cryst. Liq. Cryst. 204 (1991) 43 und 91; EP-A 0 449 015; WO 89/12039; WO 89/03821; EP-A 0 354 434 für die direkte Verknüpfung von Aromaten und Heteroaromaten;

Die Herstellung disubstituierter Pyridine, disubstituierter Pyrazine, disubstituierter Pyrimidine und disubstituierter Pyridazine findet sich beispielsweise in den entsprechenden Bänden der Serie "The Chemistry of Heterocyclic Compounds" von A. Weissberger und E. C. Taylor (Herausgeber).

Für weitere Varianten sei auch auf die Anwendungsbeispiele verwiesen.

Die erfindungsgemäßen Verbindungen eignen sich als Laserfarbstoffe.

Ein Laser ist eine lichtverstärkende Vorrichtung, die zur Erzeugung hochintensiver kohänter monochromatischer Lichtstrahlung fähig ist, welche in einem gut parallelisierten Strahl konzentriert ist, der gemeinhin als Laserstrahl bezeichnet wird. Ein typischer Laser umfaßt einen optischen Resonator mit einem laseraktiven Material, d.h. einem Lasermedium, welches ein Feststoff, eine Flüssigkeit oder ein Gas sein kann. Beim Betrieb des Lasers werden die Atome oder Moleküle des Lasermediums angeregt, bis sie Laserstrahlung aussenden, d.h. bis sie Photonen oder Lichtquanten abgeben. Zur Anregung der Atome oder Moleküle in den Laserstrahlung abgegebenen Zustand, d.h. zum Pumpen, werden sie einem Beschuß mit Elektronen oder Photonen ausgesetzt. Bei Photonenabgabe können solche Photonen die verfrühte Abgabe ähnlicher Photonen durch andere Moleküle auslösen, und zusammen bilden diese den Laserstrahl. Laser finden eine Vielfalt von Verwendungen in so verschiedenen Gebieten wie Bohren, Spektroskopie, Schweißen, Schneiden, Nachrichtenübertragung, Analysenverfahren, Chirurgie und Fotochemie.

Üblicherweise kann ein Laser nur in einem kleinen Bereich des sichtbaren Spektrums arbeiten, da er vom verwendeten Lasermedium abhängt. Dies gilt insbesondere, weil die bei einem spezifischen Energieübergang in einem gegebenen Lasermedium ausgesendeten Wellenlängen nur über einen sehr geringen Bereich des sichtbaren Spektrums einstellbar sind. Es ist daher notwendig, eine Vielfalt verschiedener Lasermedien zur Verfügung zu stellen, um Laser zum Arbeiten über den gesamten sichtbaren Spektralbereich zu befähigen.

Die vorliegende Erfindung eignet sich zum Betrieb eines Farbstofflasers, der eine Pump-Lichtquelle umfaßt, welche die Fähigkeit zur Anregung eines Farbstoffes zur Aussendung von Laserstrahlung aufweist.

Weiterhin eignet sich die vorliegene Erfindung zur Aussendung von Laserstrahlung bei verschiedenen Wellenlängen, indem ein Lasermedium einer geeigneten Pump-Lichtquelle ausgesetzt wird, das einen organischen Laser-Farbstoff gemäß dieser Erfindung beinhaltet, um das Lasermedium zur Emission von Strahlung anzuregen.

Einzelheiten zur Verwendung der erfindungsgemäßen Anmeldung finden sich auch in der europäischen Patentanmeldung mit dem Titel "Organic Solid State Light Sources With Narrow Band Width Emission" vom 04.02.1998 (Anmelder Hoechst Research and Technology GmbH & Co. KG), auf die hiermit ausdrücklich Bezug genommen wird. Sie gilt durch Zitat als Bestandteil der vorliegenden Anmeldung.

Weiterhin können die erfindungsgemäßen Verbindungen beispielsweise Verwendung finden als:
a) Elektrolumineszenzmaterialien (siehe z.B. EP-A 0 676 461)
b) Ladungstransportschicht in photovoltaischen Zellen (siehe z.B. WO-A 97/106 17) oder Strahlendetektoren (siehe z.B. DE-A 196 46 411)
c) als Werkstoffe in der nichtlinearen Optik (siehe z.B. EP-A 0 768 563)
d) als optische Aufheller (siehe z.B. DE-A 196 45 063).

Auf die zitierten Schriften wird ausdrücklich Bezug genommen, sie gelten durch Zitat als Bestandteil der vorliegenden Anmeldung.

Die Erfindung wird durch die Beispiele näher erläutert, ohne sie dadurch einzuschränken.

### Beispiele:

### A Synthesen:

### A 1 Vorstufen:

A 1.1 Synthese von 9,9'-Spirobifluoren-2-boronsäure:
(a) 2-Brom-9-(biphenyl-2-yl)-fluoren-9-ol
   2.34 g Magnesiumspäne sowie einige Kristalle lod wurden in einem mit einem Heizfön ausgeheizten und mit N₂ überlagerten 500-ml-Vierhalskolben mit Rückflußkühler und Tropftrichter vorgelegt. Nachdem ca. 20 ml trockenes THF zugegeben waren, tropfte man zunächst möglichst schnell ca. 5 ml von 22.5 g 2-Brombiphenyl zu und erhitzte an der Eintropfstelle. Nach dem Anspringen der Reaktion gab man den Rest des 2-Brombiphenyls so zu, daß das Reaktiongemisch von selbst siedete. Anschließend wurden noch etwa 180 ml THF zugegeben, bevor 2 h refluxiert wurde. Die heiße, klare Grignard-Lösung wurde in einen 1000-ml-Vierhalskolben von nicht umgesetztem Mg abdekantiert. Man kühlte auf 0°C ab und tropfte innerhalb von 20 min eine Lösung von 25.0 g 2-Bromfluoren-9-on in 290 ml THF zu. Nachdem das Eisbad entfernt worden war, rührte man bei Raumtemperatur die gelbe Suspension eine Stunde und danach 2 h unter Rückfluß. Nach dem Erkalten wurde der ausgefallene weiße Feststoff abgesaugt, gewaschen und in einer Mischung aus 180 ml Eiswasser und ca. 5 ml konz. HCI hydrolysiert. Man extrahierte mit ca. 300 ml CHCl₃. Nach Vereinigung der organischen Phasen extrahierte man zweimal mit wäßriger NaHCO₃-Lösung und anschließend mit Wasser. Nach dem Trocknen und Abziehen des Lösemittels wurde durch Umfällen aus Methylenchlorid/Hexan weiter gereinigt. Man erhielt das Produkt als weißes, kristallines Pulver.
   Ausbeute: 28.8 g (72 %).
   Schmelzpunkt: 169 - 170°C.
   1H-NMR (400 MHz, d-DMSO): δ= 8.41 - 8.39 (pdd, 1H), 7.56 - 7.51 (ptd, 1H), 7.38-7.26 (m, 3H), 7.22 - 7.17 (m, 3H), 7.14 - 7.13 (pd, 1H), 7.09 - 7.06 (m, 1H), 6.86-6.82 (m, 1H), 6.78 - 6.76 (pdd, 1H), 6.65 - 6.57 (pd, 2H), 6.23 (s, 1H), 5.95, 5.79 (2 x s, 2H).
(b) 2-Brom-9,9'-spirobifluoren
   47.0 g 2-Brom-9-(biphenyl-2-yl)-fluoren-9-ol wurden in 114 ml 99.8 %-igem Essigsäure unter Zugabe von 1.5 ml konz. HCI 2.5 h refluxiert.
   Der ausgefallene Niederschlag wurde abgesaugt und mit Wasser gewaschen. Nach dem Trockenen erhielt man das Produkt als weißes, kristallines Pulver.
   Ausbeute: 44.4 g (99%).
   Schmelzpunkt: 183°C.
   1H-NMR (400 MHz, CDCl₃): δ = 7.84 (dd, 2 H), 7.81 (dd, 1 H), 7.70 (d, 1 H), 7.49 (dd, 1 H), 7.37 (m, 3 H), 7.12 (m, 3 H), 6.85 (d, 1 H), 6.72 (m, 3 H).
(c) Synthese von 9,9'-Spirobifluoren-2-boronsäure
   1.84 g Magnesiumspäne sowie einige Kristalle lod wurden in einem ausgeheizten und mit N₂ überlagerten 2000-ml-Vierhalskolben mit Rückflußkühler und Tropftrichter vorgelegt. Man tropfte zunächst möglichst schnell ca. 25 ml einer Lösung von 30.0 g 2-Brom-9,9'-spirobifluoren in 120 ml trockenem THF zu und erhitzte an der Eintropfstelle. Nach dem Anspringen der Reaktion gab man den Rest Eduktlösung so zu, daß das Reaktionsgemisch von selbst siedet. Anschließend wurden noch ca. 1100 ml THF zugegeben, bevor 2 h refluxiert wurde. Die auf Raumtemperatur abgekühlte, klare Grignard-Lösung wurde unter N₂ in einem 2000-ml-Vierhalskolben zu einer Mischung aus 8.68 g frisch destilliertem Borsäuretrimethylester in ca. 100 ml THF innerhalb 4 h so zugetropft, daß die Innentemperatur zwischen -70 und-75°C lag. Danach wurde der Ansatz langsam auf Raumtemperatur erwärmt. Zur weißen Suspension wurden 100 g Eiswasser / 3 ml 95 - 97 %-ige H₂SO₄ gegeben. Es wurde vom nicht gelösten, anorganischen Niederschlag abgesaugt, nachgewaschen und die Mutterlauge zweimal mit wäßriger NaCI-Lösung extrahiert. Die organische Phase wurde über MgSO₄ getrocknet und das Lösemittel abgezogen. Das erhaltene Rohprodukt wurde zur Reinigung in der Wärme je zweimal mit Hexan und Acetonitril ausgerührt. Nach Trocknung erhielt man das Produkt als weißes Pulver.
   Ausbeute: 21 g (77%).
   1H-NMR (400 Mhz, d-DMSO): δ = 8.03 - 8.01 (pdd, 2H), 7.99 - 7.97 (pdd, 1H), 7.89 (s, 1H), 7.88 - 7.86 (pdd, 1H), 7.42 - 7.38 (m, 3H), 7.16 - 7.11 (m, 3H), 7.08 (ps, 1H), 6.60 - 6.59 (m, 3H).

A 1.2 Synthese 2,2',7,7'-Tetrabromspiro-9,9'-bifluoren:
Diese Verbindung wurde analog der Vorschrift in EP-A 676461 dargestellt.

A 1.3 Synthese von 2-Brom-2',7,7'-triiod-9,9'-spirobifluoren
In einem 250 ml Vierhalskolben mit Steigrohr und Tropftrichter wurde zu einer stark gerührten Lösung von 6.20 g 2-Brom-9,9'-spirobifluoren und 8.03 g lod in 35 ml CHCl₃ unter N₂ innerhalb 1.2 h eine Suspension von 14.6 g Bis-(trifluoracetoxy)iodbenzol in 70 ml CHCl₃ getropft, wobei die Innentemperatur während der Zugabe 0 - 5 °C betrug. Man ließ noch eine Stunde bei 5 - 10 °C rühren und danach über Nacht bei Raumtemperatur.
Der ausgefallene Niederschlag wurde abgesaugt. Zu diesem gab man ca. 500 ml CHCl₃ und rührte die Suspension in einer mit NaHCO₃ alkalisch gemachten wäßrigen Na₂SO₃-Lösung. Nach Abtrennung der organischen Phase wurde diese mit Wasser ausgeschüttelt und bis zur Trockene eingeengt.
Die rot gefärbte Mutterlauge wurde hintereinander mit einer wäßrigen Lösung von Na₂SO₃ und NaHCO₃ ausgeschüttelt, über Na₂SO₄ getrocknet und bis zur Trockene eingeengt.
Laut DC waren beide Fraktionen identisch. Diese versetzte man mit ca. 200 ml Aceton und ließ eine Stunde kräftig rühren. Nach Absaugen und Trocknen erhielt man das Produkt 2-Brom-2',7,7'-triiod-9,9'-spirobifluoren als weißes Pulver. Ausbeute: 9.1 g (75%).
¹H-NMR (400 MHz, CDCl₃): δ= 7.75 - 7.72 (pdt, 3H), 7.68 - 7.66 (pd, 1H), 7.57-7.52 (m, 4H), 7.00 (pt, 3H), 6.81 - 6.80 (pd, 1H).

A 1.4 Synthese von 2',7,7'-Tri-(9,9'-spirobifluoren-2-yl)-9,9'-spirobifluoren-2-boronsäure
(a) Synthese von 2-Brom-2',7,7'-tri-(9,9'-spirobifluoren-2-yl)-9,9'-spirobifluoren:
   5.00 g 2-Brom-2',7,7'-triiod-9,9'-spirobifluoren, 7.70 g 9,9'-Spirobifluoren-2-boronsäure und 5.89 g K₂CO₃ sowie 25 ml Toluol , 30 ml Wasser und 60 ml THF wurden in einem 250 ml Dreihalskolben mit Rückflußkühler vorgelegt und zur Befreiung von Sauerstoff 45 min unter N₂ bei ca. 60 °C gerührt. Anschließend gab man 370 mg Pd(PPh₃)₄ zu und ließ 48 h refluxieren.
   Nachdem das Reaktionsgemisch durch einen Faltenfilter filtriert worden war, gab man zum gelb gefärbten Filtrat 100 mg KCN und erhitzte unter Zugabe einiger Tropfen konz. wäßriger NaHCO₃-Lösung für eine Stunde bei ca. 70°C. Die Phasen wurden getrennt, wobei die wäßrige noch mit 50 ml CH₂Cl₂ extrahiert wurde. Die vereinigten organischen Phasen wurden über Na₂SO₄ getrocknet, bis zur Trockene eingeengt und getrocknet.
   Das Rohprodukt wurde mit Hexan/CH₂Cl₂ = 2 : 1 als Eluens über Kieselgel gesäult. Nach Absaugen und Trocknen erhält man das Produkt als weißes Pulver.
   Ausbeute: 5.05 g (58%)
   ¹H-NMR (400 MHz, CDCl₃): δ= 7.83 - 7.80 (pd, 6H), 7.77 - 7.75 (m, 3H), 7.71-7.65 (m, 6H), 7.63 - 7.61 (pd, 1H), 7.43 - 7.41 (dd, 1H), 7.36 - 7.29 (m, 15 H), 7.08 - 7.03 (m, 9H), 6.85 - 6.82 (m, 4H), 6.77 - 6.76 (pd, 2H), 6.73 - 6.72 (pd, 1H), 6.71 - 6.67 (m, 6H), 6.65 - 6.63 (pd, 3H).
(b) Synthese von 2',7,7'-Tri-(9,9'-spirobifluoren-2-yl)-9,9'-spirobifluorenyl-2-boronsäure
   In einem 100-ml-Dreihalskolben mit Innenthermometer und Septum tropfte man unter N₂ bei -74°C zu einer klaren Lösung von 2.00 g 2-Brom-2',7,7'-tri-(9,9'spirobifluoren-2-yl)-9,9'-spirobifluoren in 40 ml abs. THF langsam 2.8 ml einer 1.6 M Lösung von n-BuLi in Hexan innerhalb 30 Minuten zu, wobei sich das Reaktionsgemisch gelb färbte. Man ließ auf -10°C erwärmen (Grünfärbung der Lösung), kühlte wieder auf -74°C ab und gab zu der grün bleibenden klaren Lösung 620 mg Borsäuretrimethylester innerhalb 10 min zu. Danach rührte der Ansatz bei Raumtemperatur über das Wochenende (Gelbfärbung der Lösung).
   Nachdem zum gelben Reaktionsgemisch 60 ml Wasser gegeben worden waren, extrahierte man dreimal mit jeweils 30 ml CHCl₃ und die vereinigten organischen Phasen mit 30 ml Wasser. Die organische Phase wurde über Na₂SO₄ getrocknet, das Lösemittel abgezogen und über Kieselgel mit CH₂Cl₂/Hexan = 1 : 1 als Eluens gesäult, zunehmend polarer werdend (2 : 1, 3 : 1, reines CH₂Cl₂, CH₂Cl₂/MeOH, reines MeOH). Das Produkt wurde anschließend in einem Gemisch aus 50 ml Acetonitril und 25 ml Wasser bei ca. 50 °C für 30 Minuten ausgerührt. Nach Absaugen und Trocknen erhielt man das Produkt als weißes Pulver.
   Ausbeute: 1.03 g (55%).
   Da die Boronsäure ein Gemisch aus Boronsäure und verschiedenen Anhydriden darstellte, war die Interpretation des NMR-Spektrums nicht möglich. Eine kleine Probe wurde nach Verestern mit Ethylenglycol massenspektroskopisch nachgewiesen:
   MS (FD, 8 kV): *m*/*z* (%) = 1259.9 (100) [*M*⁺-B(OCH₂)₂].

A 1.5 Synthese von 2,2',7,7'-Tetrakis-(4-iodphenyl)-9,9'-spirobifluoren
In einem 250-ml-Zweihalskolben mit Rückflußkühler und Trockenrohr wurden bei Raumtemperatur zu einer Lösung von 6.00 g 2,2',7,7'-Tetraphenyl-9,9'spirobifluoren (Dargestellt nach EP-A 676461) in 100 ml CHCl₃ 4.94 g lod und 9.67 g Bis-(trifluoracetoxy)iodbenzol gegeben.
Nach 30 Minuten Rühren, wurde der ausgefallene Feststoff abgesaugt, mit CHCl₃ gewaschen, getrocknet und dreimal aus Toluol umkristallisiert. Man erhielt das Produkt 2,2',7,7'-Tetra-(4-iodphenyl)-9,9'-spirobifluoren als weißes Pulver.
Ausbeute: 2.8 g (26%).
¹H-NMR (400 MHz, CDCl₃): δ = 7.95 - 7.93 (pd, 1H), 7.63 - 7.59 (m, 3H), 7.18-7.15 (m, 2H), 6.94-6.93 (pd, 1H).

### A 2 Erfindungsgemäße Verbindungen

A 2.1 Synthese von 2,2',7,7'-Tetrakis-(9,9'-spirobifluoren-2-yl)-9,9'-spirobifluoren
967 mg 2,2',7,7'-Tetrabrom-9,9'-spirobifluoren, 2.42 g 9,9'-Spirobifluoren-2-boronsäure und 1.86 g K₂CO₃ sowie 10 ml Toluol, 15 ml Wasser und 25 ml abs. THF wurden in einem 100 ml-Dreihalskolben mit Rückflußkühler und Innenthermometer vorgelegt und zur Befreiung von Sauerstoff 45 min unter N₂ bei ca. 60°C gerührt. Anschließend gab man den Katalysator zu und refluxierte bei 75°C Innentemperatur für insgesamt 46.5 h. Nach Abkühlen auf Raumtemperatur wurde abgesaugt und der Niederschlag in ca. 30 ml CHCl₃ gelöst, die Lösung mit 10 ml konz. wäßriger NaHCO₃-Lösung versetzt und eine Lösung von ca. 400 mg KCN in 50 ml Wasser zugegeben. Anschließend wurde eine Stunde refluxiert. Nach Abtrennung der organischen Phase wurde diese getrocknet und das Lösemittel abgezogen. Das Rohprodukt wurde zweimal in Dioxan umkristallisiert. Man erhielt das Produkt als weißes Pulver.
Ausbeute: 1.60 g (67%)
Schmelzpunkt (DSC): 449°C (*T*_{g} = 273°C).
¹H-NMR ( 400 MHz, CDCl₃): δ = 7,82 - 7.80 (pd, 2H), 7.75 - 7.73 (pd, 1H), 7.66-7.64 (pq, 2H), 7.35 - 7.26 (m, 5H), 7.07 - 7.02 (m, 3H), 6.83 (pd, 1H), 6.78 - 6.77 (pd, 1H), 6.68 - 6.66 (pd, 2H), 6.64 - 6.62 (pd, 1H).

A 2.2 Synthese von 2,2',7,7'-Tetra-[4-(9,9'-spirobifluoren-2-yl)-phenyl]-9,9'spirobifluoren
1.40 g 2,2',7,7'-Tetrakis-(4-iodphenyl)-9,9'-spirobifluoren, 1.98 g 9,9'-Spirobifluoren-2-boronsäure und 1.52 g K₂CO₃ sowie 20 ml Toluol, 15 ml Wasser und 25 ml THF wurden in einem 100-ml-Dreihalskolben mit Rückflußkühler und Innenthermometer vorgelegt und zur Befreiung von Sauerstoff 45 Minuten unter N₂ bei ca. 60°C gerührt. Anschließend gab man 70 mg Pd(PPh₃)₄ zu und refluxierte für 48 Stunden. Der Verlauf der Reaktion wurde via DC kontrolliert.
Die Reaktionmischung wurde etwas eingeengt und der ausgefallene Feststoff abgesaugt, mit Wasser gewaschen und in einem Gemisch aus 30 ml CHCl₃ und 700 mg KCN in 30 ml H₂O unter Zugabe einiger Tropfen konz. wäßriger NaHCO₃-Lösung für 2.5 h refluxiert. Anschließend wurde in 50 ml Hexan für 45 Minuten refluxiert, abgesaugt, nachgewaschen und getrocknet. Schließlich wurde noch zweimal aus Dioxan umkristallisiert. Man erhielt das Produkt als weißes Pulver.
Ausbeute: 1.28 g (55%)
Schmelzpunkt (DSC): 448 °C (*T*_{g} = 272 *°C).*
¹H-NMR (400 MHz, CDCl₃): δ= 7.85 - 7.81 (m, 5H), 7.55 - 7.51 (m, 2H), 7.36 - 7.29 (m, 7H), 7.10-7.05 (m, 3H), 6.91 (pd, 1H), 6.85 (pd, 1H), 6.74 - 6.69 (m, 3H).

A 2.3 Synthese von 2,2',7,7'-Tetrakis-[(2',7,7'-tri-(9,9'-spirobifluoren-2-yl)-9,9'spirobifluoren-2-yl]-9,9'-spirobifluoren
75.0 mg 2,2',7,7'-Tetrabrom-9,9'-spirobifluoren, 690 mg 2',7,7'-Tri-(9,9'spirobifluoren-2-yl)-9,9'-spirobifluoren-2-boronsäure und 145 mg K₂CO₃ sowie 4 ml Toluol, 6 ml Wasser und 10 ml THF wurden in einem 100-ml-Zweihalskolben mit Rückflußkühler vorgelegt und zur Befreiung von Sauerstoff 45 Minuten unter N₂ bei Raumtemperatur gerührt. Anschließend gab man 2.1 mg Pd(PPh₃)₄ zu und ließ bei 75 °C Innentemperatur für insgesamt 140 Stunden refluxieren.
Zur zweiphasigen Reaktionsmischung gab man eine Lösung von 60 mg KCN in 30 ml H₂O zu und erhitzte für 1 h bei ca. 50 °C. Nach Abtrennung der organischen Phase wurde diese mit 100 ml MeOH gefällt, der ausgefallene Feststoff abgesaugt, getrocknet und über Kieselgel mit Hexan/CH₂Cl₂ = 1 : 1 als Laufmittel gesäult. Das Produkt wurde als weißes Pulver nach zweimaliger Umkristallisation in Dioxan erhalten.
Ausbeute: 200 mg (31%).
Da das komplexe H-NMR keine genaue Strukturbestimmung zuließ, wurde die Verbindung via hochaufgelöster Masse analysiert.
MS (MALDI-TOF): *m*/*z* = 5346.63 [*M*⁺]. Theoretischer Wert: 5346.65

### B Verwendung der erfindungsgemäßen Verbindungen in Emissionsvorrichtungen

Von den Verbindungen A 2.1 bis A 2.3 wurden dünne (100 nm) amorphe Filme erzeugt. Dies geschah durch Spin-Coating von Lösungen der jeweiligen Verbindungen in Chlorbenzol. Dazu wurden die Verbindungen zunächst vollständig gelöst (10 mg/ml; die Lösungen wurden erzeugt, indem die Verbindungen für ca. 15 Stunden bei 50°C unter N₂ gerührt wurden) und anschließend bei geeigneten Umdrehungsgeschwindigkeiten aufgeschleudert.

Dabei wurde bei allen drei Verbindungen brilliante blaue Photolumineszenz mit extrem hoher Effizienz erhalten. Diese Verbindungen weisen nach unserem Wissen die höchsten jemals berichteten PL-Effizienzen im festen, unverdünnten Zustand auf:

| Verbindung | λ_{PL-film} [nm] | Φ_{PL-film} [%] |
|---|---|---|
| A 2.1 | 403, 425 | >90 |
| A 2.2 | 414, 435 | >90 |
| A 2.3 | 425, 447 | >90 |

Desweiteren wurden mit den Verbindungen Experimente hinsichtlich ihrer Eigenschaften als Laserfarbstoffe (amplified sponteous emission = ASE; verstärkte spontane Emission) gemacht.
Dazu wurden die erzeugten Filme optisch gepumpt (gepulster Stickstofflaser; Anregung bei 337 nm) und die Linienverengung in Abhängigkeit von der Anregungsintensität verfolgt. Hier ergaben sich folgende Ergebnisse. Diese Ergebnisse wurde verglichen mit denjenigen von zwei nicht erfindungsgemäßen Verbindungen.

| | Beginn deutlicher | Linienbreite der Emission bei halber maximaler Höhe |
|---|---|---|
| Verbindung | Linienverengung [µJ/cm²] | [nm]³⁾ |
| A 2.1 | 2 | 3.5 |
| A 2.2 | 2 | 3.1 |
| A 2.3 | 1.3 | 2.3 |
| Spiro-6-PP¹⁾ | 4 | 8 |
| Spiro-4-PP²⁾ | 30 | 46 |

| | | |
|---|---|---|
| ¹⁾ 2,2',7,7'-Tetrakis(4-biphenyl)-9,9'-spirobifluoren; | | |
| ²⁾ 2,2',7,7'-Tetraphenyl-9,9'-spirobifluoren. | | |
| ³⁾ Anregungsintensität: 10 µJ/cm² | | |

Diese Messungen belegen, daß die erfindungsgemäßen Verbindungen bei extrem niedrigen Anregungsenergien bereits das Phänomen der ASE zeigen. Dies prädestiniert die erfindungsgemäßen Verbindungen zur Verwendung in optisch gepumpten Lasern. Elektrisch gepumpte organische Laser sind derzeit noch nicht verfügbar; sollten diese jedoch entwickelt werden, sind die erfindungsgemäßen Verbindungen wegen ihrer extrem hohen PL-Effizienz im festen - unverdünnten-Zustand und ihrer hohen Thermostabilität bestens dafür geeignet.

## Patentansprüche

1. Verbindungen der Formel (I) 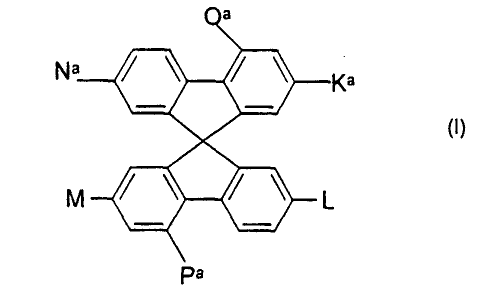 wobei K^{a}, L, M, N^{a}, O^{a}, P^{a} unabhängig voneinander gleich oder verschieden, 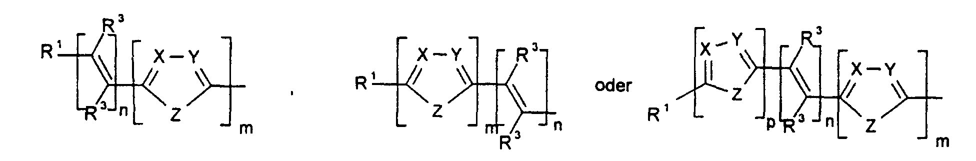 sind;
wobei die Symbole und Indizes die folgenden Bedeutungen haben:
R³ H, C₁-C₂₂-Alkyl, CN, C₆-C₁₄-Aryl, C₄-C₁₅-Heteroaryl, C₅-C₂₀-Alkylaryl und C₅-C₂₀-Arylalkyl
m, n, p: unabhängig voneinander gleich oder verschieden 0, 1, 2, 3, 4, 5, oder 6;
X, Y: CR, N
Z: O, S, NR, CR₂, -CH=CH-, -CH=N-, -CR²=CR²- oder -CR²=N-;
R : H, C₁-C₂₂-Alkyl (linear, verzweigt oder cyclisch), C₁-C₂₂-Alkoxy, CN, NR²₂, COOR², CHO, SR², NO₂, OH, C₆-C₁₄-Aryl, wie Phenyl, Biphenyl, 1-Naphthyl, 2-Naphthyl, 9-Anthryl, C₄-C₁₅-Heteroaryl oder Aryloxy,
R¹: unabhängig voneinander, gleich oder verschieden R, A, B oder C ,
wobei 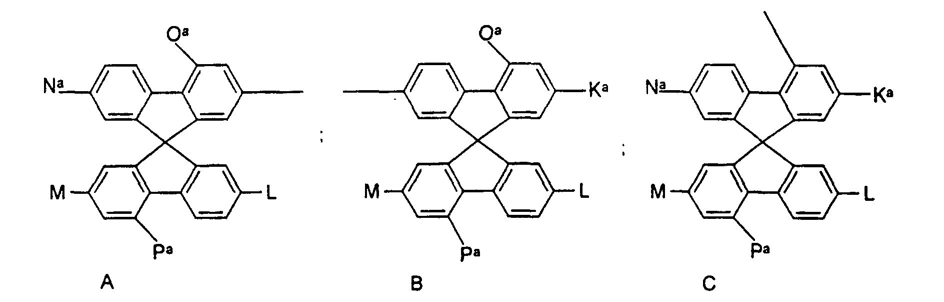 wobei mindestens ein Rest R¹: A, B oder C ist;
R² ist gleich oder verschieden H oder ein Kohlenwasserstoffrest mit 1 bis 30 C-Atomen, der auch ein oder mehrere Heteroatome, enthalten kann,
ausgenommen Verbindungen der Formel (I) bei denen
| | |
|---|---|
| bei O^{a}, P^{a}, M und L gleichzeitig | R¹ ungleich A, B oder C ist |
| und zugleich bei K^{a} und N^{a} | R¹ gleich A oder B bedeutet. |
sowie Verbindungen der Formel (I) bei denen
| | |
|---|---|
| bei O^{a}, K^{a}, M und P^{a} gleichzeitig | R¹ ungleich A, B oder C ist |
| und zugleich bei N^{a} und L | R¹ gleich A oder B bedeutet. |

2. Verbindungen gemäß Anspruch 1, **dadurch gekennzeichnet, daß** die Symbole und Indizes die folgenden Bedeutungen haben:
n, p : 0;
m : 0, 1, 2, 3, 4, 5 oder 6;
X, Y : CR;
Z : -CR=CR-; und
R¹ : unabhängig voneinander gleich oder verschieden R bzw. A, B oder C, wobei mindestens ein R¹: A, B oder C ist.

3. Verbindungen gemäß Anspruch 1 oder 2, **dadurch gekennzeichnet, daß** in Formel I für K^{a}, L, M, N^{a}: R¹ unabhängig voneinander gleich oder verschieden A, B oder C ist.

4. Verbindungen gemäß mindestens einem der vorhergehenden Ansprüche, **dadurch gekennzeichnet, daß** in Formel (I) O^{a} und P^{a} gleich H ist.

5. Verbindungen gemäß Anspruch 1 oder 2, **dadurch gekennzeichnet, daß** in Formel (I) R¹ gleich oder verschieden A, B oder C ist.

6. Verbindungen gemäß mindestens einem der vorhergehenden Ansprüche, **dadurch gekennzeichnet, daß** R¹: R oder A ist, wobei mindestens ein R¹ gleich A ist.

7. Verbindungen gemäß mindestens einem der vorhergehenden Ansprüche, **dadurch gekennzeichnet, daß** R¹ gleich A ist.

8. Verbindungen gemäß Anspruch 1 der Formel (II) 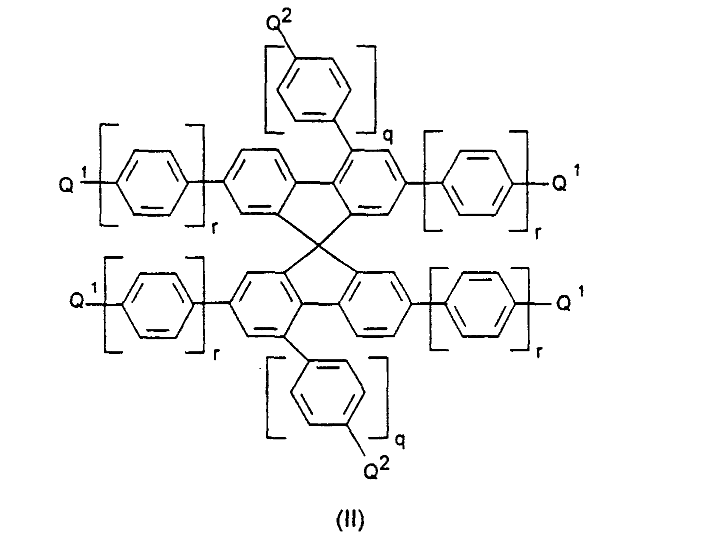 wobei die Symbole und Indizes die folgenden Bedeutungen haben:
q, r : unabhängig voneinander gleich oder verschieden 0, 1, 2, 3, 4, 5 oder 6;
Q¹, Q²: unabhängig voneinander gleich oder verschieden H oder 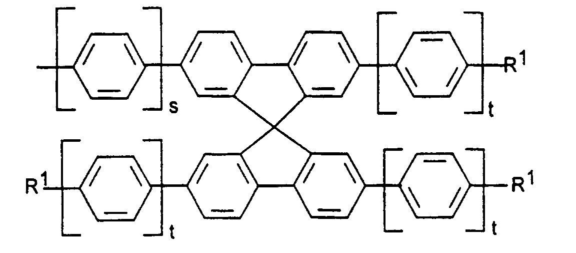
wobei
s,t: unabhängig voneinander gleich oder verschieden 0, 1, 2, 3 oder 4 ist; und
R¹: unabhängig voneinander gleich oder verschieden R bzw. A, B oder C ist, und mindestens ein Rest Q¹ and Q² ungleich wasserstoff ist.

9. Verbindungen gemäß Anspruch 8, **dadurch gekennzeichnet, daß** die Symbole und Indizes die folgenden Bedeutungen haben:
Q¹, Q²: unabhängig voneinander gleich oder verschieden H oder 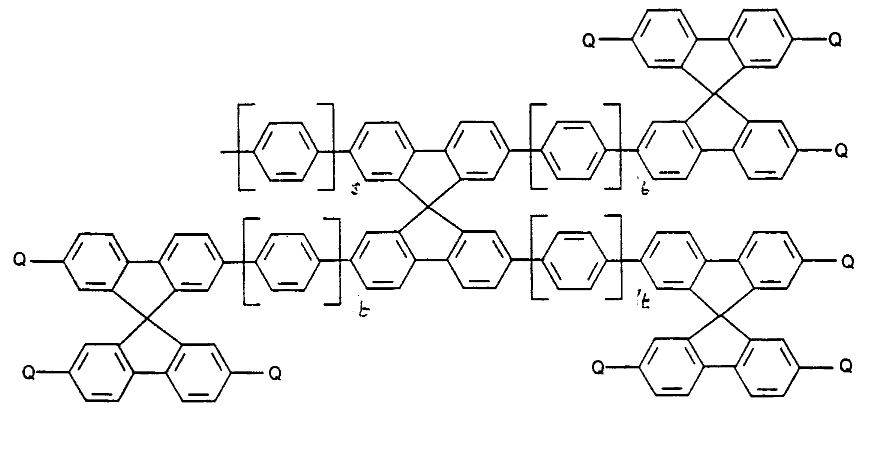
wobei
s, t: unabhängig voneinander gleich oder verschieden 0, 1, 2, 3 oder 4 ist; und
Q : unabhängig voneinander gleich oder verschieden R oder Q¹ ist.

## Claims

1. A compound of the formula (I) 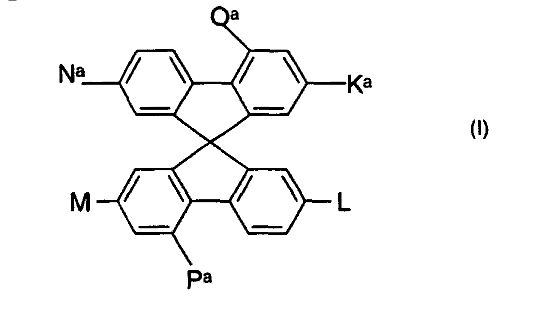 where Ka, L, M, Na, Oa and Pa, independently of one another, are identical or different and are 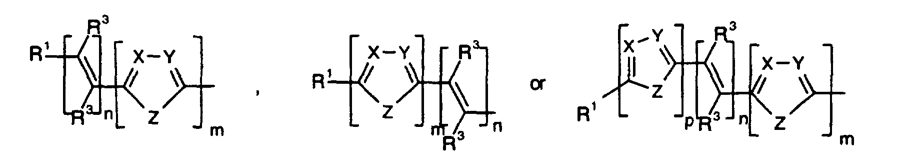 where the symbols and indices have the following meaning:
R³ H, C₁-C₂₂-alkyl, CN, C₆-C₁₄-aryl, C₄-C₁₅-heteroaryl, C₅-C₂₀-alkylaryl or C₅-C₂₀-arylalkyl
m, n, and p: independently of one another, identically or differently, 0, 1, 2, 3, 4, 5 or 6;
X and Y: CR or N
Z: O, S, NR, CR², -CH=CH-, -CH=N-, -CR²=CR²- or -CR²=N-;
R: H, C₁-C₂₂-alkyl (linear, branched or cyclic), C₁-C₂₂-alkoxy, CN, NR²₂, COOR², CHO, SR², NO2, OH, C₆-C₁₄- aryl, such as phenyl, biphenyl, 1-naphthyl, 2-naphthyl or 9-anthryl, or C₄-C₁₅-heteroaryl or aryloxy,
R¹: independently of one another, identically or differently, R, A, B or C , where 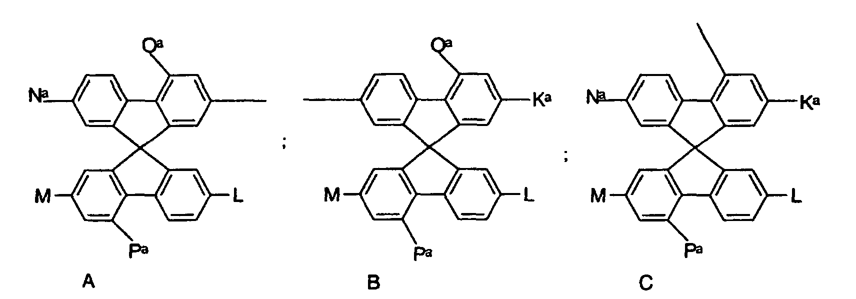
where at least one radical R¹ is A, B or C;
R² is, identically or differently, H or a hydrocarbon radical having 1 to 30 carbon atoms, which may also contain one or more heteroatoms,
with the exception of compounds of the formula (I)
in which
| | |
|---|---|
| in O^{a}, P^{a}, M and L simultaneously | R¹ is not A, B or C |
| and at the, same time in K^{a} and N^{a} | R¹ is A or B |
and compounds of the formula (I)
in which
| | |
|---|---|
| in O^{a}, K^{a}, M and P^{a} simultaneously | R¹ is not A, B or C |
| and at the same time in Na and L | R¹ is A or B. |

2. A compound as claimed in claim 1, wherein the symbols and indices have the following meanings:
n and p : 0;
m : 0, 1, 2, 3, 4, 5 or 6;
X and Y : CR;
Z : -CR=CR-; and
R¹ : independently of one another, identically or differently, R or A, B or C, where at least one R¹ is A, B or C.

3. A compound as claimed in claim 1 or 2, wherein R¹ for K^{a}, L, M and N^{a} in the formula I is, independently of one another, identically or differently, A, B or C.

4. A compound as claimed in at least one of the preceding claims, wherein O^{a} and P^{a} in the formula (I) are H.

5. A compound as claimed in claim 1 or 2, wherein R¹ in the formula (I) is, identically or differently, A, B or C.

6. A compound as claimed in at least one of the preceding claims, wherein R¹ is R or A, where at least one R¹ is A.

7. A compound as claimed in at least one of the preceding claims, wherein R¹ is A.

8. A compound as claimed in claim 1 of the formula (II) 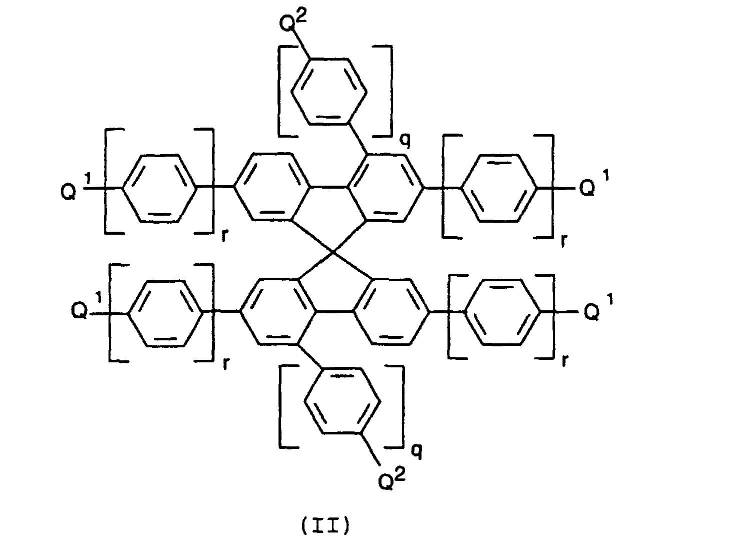 where the symbols and indices have the following meanings:
q and r: independently of one another, identically or differently, 0, 1, 2, 3, 4, 5 or 6;
Q¹ and Q²: independently of one another, identically or differently, H or 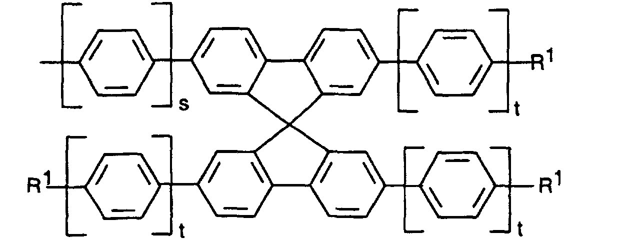
where
s and t: independently of one another, identically or differently, is 0, 1, 2, 3 or 4; and
R¹: independently of one another, identically or differently, is R or A, B or C and at least one of Q¹ and Q² is not hydrogen.

9. A compound as claimed in claim 8, wherein the symbols and indices have the following meanings:
Q¹ and Q²: independently of one another, identically or differently, H or 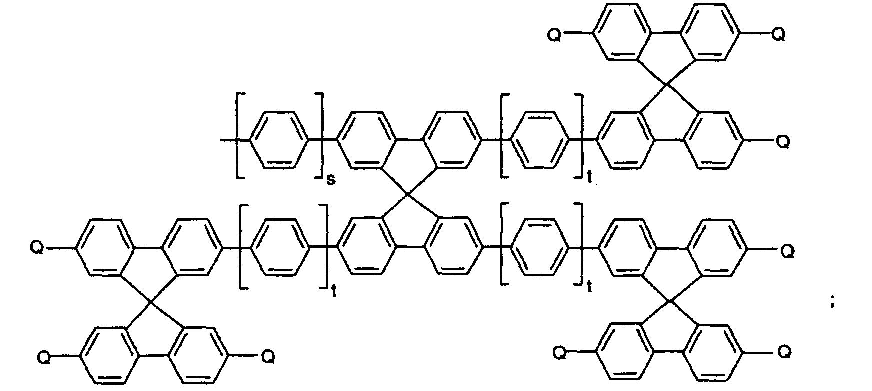
where
s and t: independently of one another, identically or differently, are 0, 1, 2, 3 or 4; and
Q : independently of one another, identically or differently, is R or Q¹.

## Revendications

1. Composés de formule 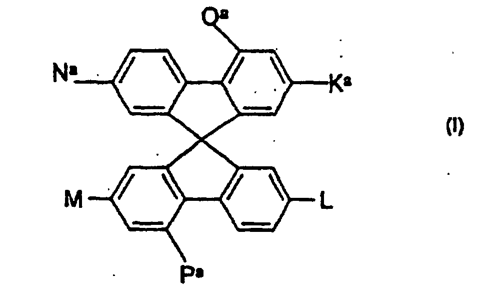 où K^{a}, L, M, N^{a}, O^{a}, P^{a} sont indépendamment l'un de l'autre identiques ou différents 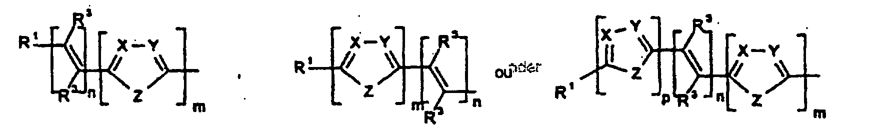 les symboles et les indices possédant les significations suivantes :
R³ représente un atome d'hydrogène, un groupe alkyle en C₁-C₂₂, CN, aryle en C₆-C₁₄, hétéroaryle en C₄- C₁₅, alkylaryle en C₅-C₂₀ et arylalkyle en C₅-C₂₀,
m, n, p : valent indépendamment, identiques ou différents, 0, 1, 2, 3, 4, 5 ou 6 ;
X, Y représente des groupes CR, N,
Z représente un groupe O, S, NR, CR₂, -CH=CH-, -CH=N-, -CR²-CR² ou -CR²=N- ;
R représente un atome d'hydrogène, un groupe alkyle en C₁-C₂₂ (linéaire, ramifié ou cyclique), alkoxy en C₁-C₂₂, CN, NR²₂, COOR², CHO, SR², NO₂, OH, aryle en C₆-C₁₄ comme phényle, biphényle, 1-naphtyle, 2- naphtyle, 9-anthryle, hétéroaryle en C₄-C₁₅ ou aryloxy,
R¹ représentent, indépendamment les uns des autres, identiques ou différents, R, A, B ou C, où 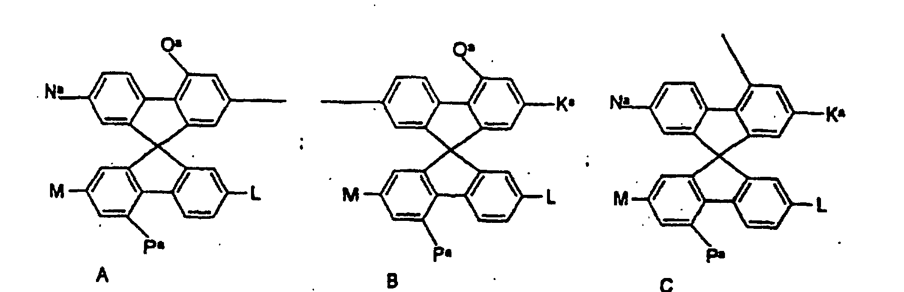
au moins un reste R¹ : représente A, B ou C ;
R² représentent, identiques ou différents, un atome d'hydrogène ou un reste hydrocarboné présentant 1 à 30 atomes de carbone, qui également peut contenir un ou plusieurs hétéroatomes,
à l'exception de composés de formule (I) dans lesquels
| | |
|---|---|
| pour O^{a}, P^{a}, M et L simultanément | R¹ ne représente pas A, B ou C |
| et en même temps pour K^{a} et N^{a} | R¹ représente A ou B. |
ainsi que des composés de formule (I) pour lesquels
| | |
|---|---|
| pour O^{a}, K^{a}, M et P^{a} simultanément | R¹ ne représente pas A, B ou C |
| et en même temps pour N^{a} et L | R¹ représente A ou B. |

2. Composés selon la revendication 1, **caractérisés en ce que** les symboles et les indices possèdent les significations suivantes :
n, p valent 0 ;
m vaut 0, 1, 2, 3, 4, 5 ou 6 ;
X, Y représentent un groupe CR ;
Z représente -CR=CR- ; et
R¹ représentent, indépendamment l'un de l'autre, identiques ou différents, R ou A, B ou C, au moins un R¹ représente A, B ou C.

3. Composés selon la revendication 1 ou 2, **caractérisés en ce que** dans la formule I pour K^{a}, L, M, N^{a} : R¹ représentent indépendamment les uns des autres, identiques ou différents, A, B ou C.

4. Composés selon au moins l'une des revendications précédentes, **caractérisés en ce que**, à la formule (I), O^{a} et P^{a} représentent H.

5. Composés selon la revendication 1 ou 2, **caractérisés en ce que**, à la formule (I), R¹ sont identiques ou différents et représentent A, B ou C.

6. Composés selon au moins l'une des revendications précédentes, **caractérisés en ce que** R¹ : R ou A, au moins un R¹ étant égal à A.

7. Composés selon au moins l'une des revendications précédentes, **caractérisés en ce que** R¹ représente A.

8. Composés selon la revendication 1 de formule (II) 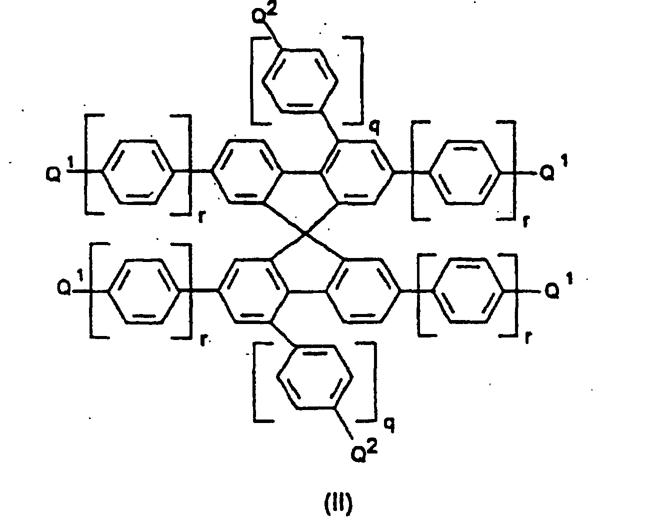 les symboles et les indices possèdent les significations suivantes :
q, r valent, indépendamment l'un de l'autre, identique ou différent, 0, 1, 2, 3, 4, 5 ou 6 ;
Q¹, Q² représentent, indépendamment l'un de l'autre, identiques ou différents, un atome d'hydrogène ou un groupe de formule 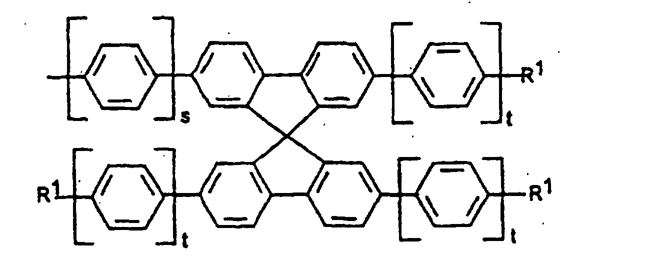
où
s, t valent, indépendamment l'un de l'autre, identiques ou différents, 0, 1, 2, 3 ou 4 ; et
R¹ représentent, indépendamment les uns des autres, R ou A, B ou C, et au moins un reste Q¹ ou Q² ne représentent pas un atome d'hydrogène.

9. Composés selon la revendication 8, **caractérisés en ce que** les symboles et les indices possèdent les significations suivantes :
Q¹, Q² représentent, indépendamment l'un de l'autre, identiques ou différents, un atome d'hydrogène ou groupe de formule 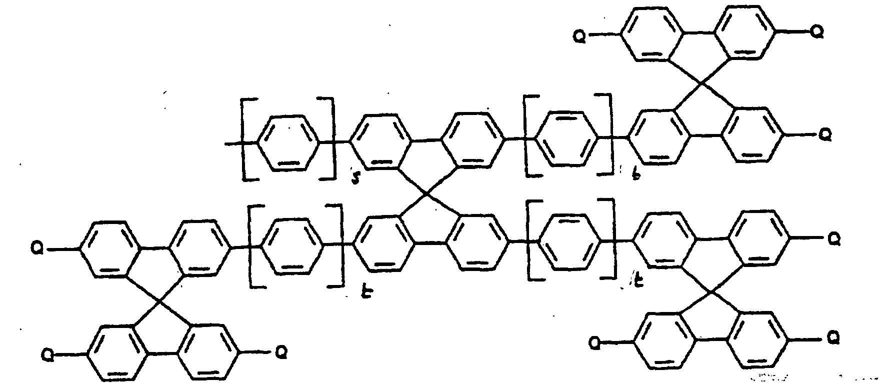
où
s, t valent, indépendamment l'un de l'autre, identiques ou différents, 0, 1, 2, 3 ou 4 ; et
Q représentent, indépendamment les uns des autres, identiques ou différents, R ou Q¹.
